# EUROPÄISCHE PATENTANMELDUNG

(11) **EP 4 445 992 A1**
(43) Veröffentlichungstag der Anmeldung: **16.10.2024**
(21) Anmeldenummer: 24166738.5
(22) Anmeldetag: 27.03.2024
(51) Int. Cl.: B01D 61/02, B01D 61/12, C02F 1/44

(54) **MEHRSTUFIGE MEMBRANFILTRATIONSANLAGE UND VERFAHREN ZUM BETREIBEN EINER MEHRSTUFIGEN MEMBRANFILTRATIONSANLAGE**

(30) Priorität: 05.04.2023 DE 102023108726
(71) Anmelder: B. Braun Avitum AG, 34212 Melsungen (DE)
(72) Erfinder: Lieb, Nino, 34212 Melsungen (DE); Odernheimer, Philipp, 34212 Melsungen (DE); Giesa, Jonas, 34121 Kassel (DE); Krietemeyer, Stephan, 79400 Kandern (DE)
(74) Vertreter: Tergau & Walkenhorst Intellectual Property GmbH

(57) **Zusammenfassung**

Membranfiltrationsanlage (100), insbesondere zur Umkehrosmose, umfassend eine Mehrzahl von Stufen (102, 104, 190), wobei jede Stufe (102, 104, 190) wenigstens eine Pumpe und eine Filtermembran umfasst, und wobei die Stufen (102, 104, 190) hydraulisch in Folge durch Verbindungsleitungen (103, 191, 192) hydraulisch miteinander verbunden sind, und wobei für jede Stufe (102, 104, 190) eine Umgehungsleitung (110, 111) zur Umleitung des Flüssigkeitsstroms um diese Stufe (102, 104, 190) vorgesehen ist, und wobei wenigstens ein Umgehungsventil (107, 108, 170, 207, 208, 209) vorgesehen ist, welches im geschlossenen Zustand die jeweilige Umgehungsleitung (110, 111) sperrt, wobei eine Steuer- und Regeleinheit (109) vorgesehen ist, welche konfiguriert ist, das jeweilige Umgehungsventil (107, 108, 170, 207, 208, 209) anzusteuern.

## Beschreibung

Die Erfindung betrifft Membranfiltrationsanlage, insbesondere zur Umkehrosmose, umfassend eine Mehrzahl von Stufen, wobei jede Stufe wenigstens eine Pumpe und eine Filtermembran umfasst, und wobei die Stufen hydraulisch in Folge durch Verbindungsleitungen hydraulisch miteinander verbunden sind, und wobei für jede Stufe eine Umgehungsleitung zur Umleitung des Flüssigkeitsstroms um diese Stufe vorgesehen ist, und wobei wenigstens ein Umgehungsventil vorgesehen ist, welches im geschlossenen Zustand die jeweilige Umgehungsleitung sperrt, Sie betrifft weiterhin ein Verfahren zum Betreiben der Membranfiltrationsanlage.

Bei bekannten mehrstufigen Membranfiltrationsanlagen mit mehreren Stufen, die jeweils wenigstens eine Filtermembran und wenigstens eine Pumpe zum Pumpen von Flüssigkeit durch diese Filtermembran umfassen, wird das Permeat derjeweils vorherigen Stufe in die jeweils darauffolgende Stufe geleitet. Wenn eine Stufe vollständig ausfällt oder nicht funktionsfähig ist, kann durch eine manuelle Betätigung eines entsprechenden Ventils der Flüssigkeitsstrom direkt und unter Umgehung der defekten Stufe in die nächste Stufe geleitet werden.

Nachteilig bei den beschriebenen bekannten Membranfiltrationsanlagen bzw. Membranfilteranlagen ist, dass das entsprechende Ventil manuell von Hand betätigt werden muss. Bis zum Umschalten des oder der Ventile kann eine Versorgung der angeschlossenen Verbraucher nicht gewährleistet werden. Durch falsche Ventilstellungen kann ungefiltertes Wasser in die Ringleitung strömen, welches z. B. bei der Dialysetherapie zu einer Gefährdung der Patienten führen kann.

Der Erfindung liegt daher die Aufgabe zugrunde, eine Membranfiltrationsanlage dahingehend zu verbessern, dass bei Ausfall einer Stufe die Versorgung der angeschlossenen Verbraucher sichergestellt wird und die Deaktivierung der ausgefallen Stufe zuverlässig erfolgt. Weiterhin soll ein verbessertes Verfahren zur Membranfiltration angegeben werden.

In Bezug auf die Membranfiltrationsanlage wird diese Aufgabe erfindungsgemäß dadurch gelöst, dass eine Steuer- und Regeleinheit vorgesehen ist, welche konfiguriert ist, das jeweilige Umgehungsventil anzusteuern.

Vorteilhafte Ausgestaltungen der Erfindung sind Gegenstand der Unteransprüche.

Die Erfindung geht von der Überlegung aus, dass bei mehrstufigen Anlagen bei Beschädigung bzw. Ausfall einer Stufe die Versorgung der Verbraucher möglichst unterbrechungsfrei sichergestellt werden sollte. Dies ist besonders wichtig beim Einsatz der Anlage bei der Dialysetherapie.

Wie nunmehr erkannt wurde, können diese Anforderungen erfüllt werden, indem bei mehrstufigen Membranfiltersystemen, welche das Permeat mehrfach filtern, ein Ausfall einer Filterstufe durch das Einleiten eines Notbetriebs kompensiert wird. Dafür wird der defekte bzw. nicht funktionsfähige Teil der Anlage (hydraulische Komponenten und/oder Sensoren) durch eine Flussumleitung innerhalb des Membranfiltersystems bzw. der Membranfiltrationsanlage gebypasst.

Die Vorteile der Nutzung einer doppelstufigen Anlage sind dabei im Wesentlichen, die Ausfallsicherheit und auch regulatorische Anforderungen (manche Länder fordern doppelt filtriertes Wasser für die Verwendung in der Hämodialyse).

Der Ausdruck, dass die Steuer- und Regeleinheit konfiguriert ist, die Umgehungsventile anzusteuern, bedeutet dabei insbesondere, dass die Steuer- und Regeleinheit angepasst bzw. konzipiert bzw. programmiert bzw. eingerichtet ist, die Ansteuerung durchzuführen. Die entsprechende Ansteuerung ist dabei vorzugsweise hardware- und/oder softwaremäßig in der Steuer- und Regeleinheit implementiert.

Vorteilhafterweise ist die Steuer- und Regeleinheit konfiguriert, in einem Notbetrieb mit wenigstens einer defekten Stufe das der jeweiligen Stufe zugeordnete Umgehungsventil zu öffnen, sodass die Flüssigkeit um die defekte Stufe herumgeleitet wird. Die Umgehungsleitung führt die Flüssigkeit jeweils an der defekten Stufe vorbei zur hydraulisch sich anschließenden bzw. nächsten Stufe.

In einer bevorzugten Ausführungsform ist in jeder Umgehungsleitung ein Umgehungsventil angeordnet. Bei einer zweistufigen Anlage sind somit zwei Umgehungsleitungen vorgesehen, in denen jeweils ein Umgehungsventil angeordnet ist.

Vorteilhafterweise ist in der Umgehungsleitung jeweils ein Rückschlagventil angeordnet, insbesondere dem jeweiligen Umgehungsventil in der Umgehungsleitung stromaufwärts nachgeschaltet, welches den Rückfluss durch das Umgehungsventil sperrt. Rückschlagventile sind besonders gut zum Vermeiden der Rückflüsse geeignet, da diese durch den einfachen mechanischen Aufbau langlebig und fehlerunanfällig sind. Außerdem benötigen diese Ventile keine Steuerungslogik, da der einsetzende Notbetrieb die Druckverhältnisse innerhalb der Anlage in einer Weise umkehren, sodass die Rückschlagventile von selbst schließen. Als Alternative zu Rückschlagventilen können ebenfalls elektrisch angesteuerte Ventile verwendet werden, welche mit der genannten Steuer- und Regeleinheit verbunden werden.

In einer bevorzugten Ausführungsform ist wenigstens ein Umgehungsventil als Mehrwegeventil ausgebildet. Bei einer zweistufigen Membranfiltrationsanlage wird auf diese Weise nur ein Ventil benötigt, um bedarfsweise die erste oder die zweite Stufe hydraulisch zu überbrücken. Es wird auch nur eine Signalverbindung von der Steuer- und Regeleinheit zu dem Mehrwegeventil benötigt, sodass Material gespart wird und die Konfiguration der Anlage vereinfacht wird.

Vorteilhafterweise ist die Steuer- und Regeleinheit konfiguriert, signaleingangsseitig Sensordaten und/oder Prozessdaten der Membranfiltrationsanlage zu empfangen. Auf diese Weise wird die Steuer- und Regeleinheit ertüchtigt, die Anlage zu überwachen und Fehlfunktionen, insbesondere den Ausfall/Defekt oder Funktionsstörung einer Stufe zu erkennen und darauf zu reagieren. Die Steuerungseinheit des Membranfiltersystemen erkennt die Störung und leitet den Notbetrieb automatisch ein, wodurch Anlagenstillstandzeiten vermieden und falsche Nutzerinteraktionen (z. B. Ventilfehlstellungen) präventiv verhindert werden.

Die Steuer- und Regeleinheit öffnet dabei in einem Notbetrieb das der jeweiligen Stufe zugeordnete Umgehungsventil, sodass die Flüssigkeit um die defekte Stufe herumgeleitet wird, wenn eine die empfangenen Sensordaten und/oder Prozessdaten außerhalb eines vorgegebenen Schwellenbereiches liegen, insbesondere wenn ein Null-Signal eines Sensors vorliegt.

Die Sensoren können kontinuierlich überwacht werden, wenn diese ein "Minimalsignal" (z. B. 4 mA) aussenden, um den Unterschied zwischen dem gemessenen 0-Wert und einem Defekt zu ermöglichen. Die Sensoren und/oder Prozesswerte können mittels logischer Statements auf Funktionalität geprüft werden. Beispielhaft: WENN Betriebsmodus "An" DANN Volumenstrom Stufe 1 GRÖSSER 300l/h. Sollte diese Bedingung nicht mehr wahr sein, kann ein Defekt der ersten Stufe getriggert werden. Das logische Statement ist beispielhaft und kann beliebig mit logisch verknüpften Sensorkombinationen und Prozesswerten erweitert werden. Diese Erweiterung kann durch Logik-Operatoren (UND/ ODER /NICHT etc.) durchgeführt werden.

Die Membranfiltrationsanlage umfasst dazu bevorzugt wenigstens einen Drucksensor und/oder wenigstens einen Volumenstromsensor bzw. Durchflusssensor, wobei der jeweilige Sensor zur Übertragung von Sensordaten signaleingangsseitig mit der Steuer- und Regeleinheit verbunden ist. Auf diese Weise können die Zustände der Komponenten der Anlage erfasst werden. Bevorzugt umfasst jede der Stufen einen Drucksensor und/oder wenigstens einen Volumenstromsensor bzw. Durchflusssensor, sodass für jede der Stufen separat der Druck und Volumenstrom erfasst werden kann, wodurch das gezielte Detektieren eines Ausfalls bzw. einer Fehlfunktion einer Stufe verbessert wird. Neben dem Erfassen und Auswerten von Sensordaten können auch binäre Signale ausgewertet werden, um daraus Rückschlusse auf größere Ausfälle zu ziehen. Zum Beispiel können Sicherungen ausgewertet werden und das Auslösen von Sicherungen kann auf den Ausfall von Stufen oder einzelnen Pumpen korreliert werden.

Der wenigstens eine Drucksensor ist bevorzugt ein Drucksensor zum Messen des Füllstands eines Tanks, insbesondere des Vorhaltetanks der Membranfiltrationsanlage, und/oder der wenigstens eine Volumenstromsensor ist bevorzugt ein Volumenstromsensor zum Messen des Konzentratstroms und/oder der wenigstens eine Volumenstromsensor ist ein Volumenstromsensor zum Messen des Permeatstroms ist.

Die Membranfiltrationsanlage umfasst bevorzugt wenigstens eine Pumpe, eine wenigstens eine Filtermembran und wenigstens einen Sicherungsautomat und/oder wenigstens einen Schutzschalter, welche/r zur Übertragung von Prozessdaten signaleingangsseitig mit der Steuer- und Regeleinheit verbunden ist. Die Membranfiltrationsanlage umfasst zwei oder mehr Stufen. Die jeweilige Stufe umfasst dabei bevorzugt eine Druckerhöhungspumpe, welche die in die Stufe in einem Zulaufstrom geleitete Flüssigkeit in ein Membranmodul presst. Das Membranmodul umfasst eine oder mehrere Membranen, die jeweils in Gruppen in Reihe oder parallelgeschaltet sind. In einer Stufe können mehrere Membranmodule in Reihe zueinander geschaltet werden.

Durch den Prozess entstehen ausgehend von dem Membranmodul in einer Permeatleitung ein filtrierter Permeatstrom und in einer Konzentratleitung ein Konzentratstrom, welcher die zurückgehaltenen Stoffe abtransportiert. Die jeweilige Stufe umfasst daher bevorzugt eine Zirkulationspumpe, welche einen Teil des Konzentrats, d. h. das rezirkulierte Konzentrat, in einem Konzentratstroms zurück zur Druckerhöhungspumpe fördert, wo es sich mit dem Zulaufstrom vermischt und als Feedwasser den Prozess wiederholt. Der nicht rezirkulierte Anteil des Konzentrats wird über ein Ventil (z. B. ein Magnetventil, Nadelventil, elektrisches Stellventil, o. Ä.) aus der Membranfiltrationsanlage ausgeleitet.

Die Steuer- und Regeleinheit, kann, insbesondere zusätzlich zu den Druck- und Volumenverhältnissen, Informationen über den Betriebszustand wichtiger Komponenten erhalten und auswerten, wodurch Genauigkeit und Zuverlässigkeit des Erkennens bzw. Detektierens einer ausgefallenen oder nicht ordnungsgemäß funktionierenden Stufe verbessert werden.

Die signaleingangsseitige Verbindung, d. h. die Verbindung zwischen Sensoren und/oder Frequenzumrichtern und/oder Sicherungsautomaten und/oder Schutzschaltern zur Steuer- und Regeleinheit kann dabei jeweils analog, digital über elektrische Übertragungsleitungen und/oder über einen Bus erfolgt.

In einer bevorzugten Ausführungsform öffnet die Steuer- und Regeleinheit bei einem Über- oder Unterschreiten wenigstens eines Schwellenwertes der Sensor- und/der Prozessdaten in einem Notbetrieb wenigstens ein Umgehungsventil. Die Steuer- und Regeleinheit ordnet dabei insbesondere diese Daten jeweils einer oder mehrerer Stufen zu, welche damit als defekt oder nicht funktionsfähig erkannt und abgeschaltet werden. Durch das des jeweiligen Umgehungsventils wird die entsprechende Stufe hydraulisch überbrückt. Bei mehrstufigen Anlagen mit drei oder mehr Stufen können mehrere Umgehungsventile geöffnet werden, sodass die entsprechenden Stufen hydraulisch überbrückt werden.

Vorteilhafterweise steuert die Steuer- und Regeleineit im Notbetrieb wenigstens eine Pumpe derart an, dass weiterhin Prozesseingangswasser durch wenigstens ein intaktes Membranmodul gepresst wird. Dadurch wird sichergestellt, dass bei Ausfall einer Stufe die Verbrauer auch weiterhin mit Permeat versorgt werden können.

Die Steuer- und Regeleinheit ist vorteilhafterweise konfiguriert, die Komponenten der Membranfiltrationsanlage, welche kontinuierliche Signale ausgeben, insbesondere Drucksensoren und/oder Volumenstromsensoren, dauerhaft zu überwachen und die Komponenten der Membranfiltrationsanlage, welche keine kontinuierlichen Signale ausgeben, insbesondere Magnetventile, in einem Komponententest zu prüfen. Der Komponententest erzeugt Stimuli in der Anlage, welche ein erwartetes Ergebnis erfüllen müssen. Sollte dieses Ergebnis nicht den definierten Erwartungen entsprechen wird die getestete Komponente als defekt angesehen.

Die Steuer- und Regeleinheit ist vorzugsweise konfiguriert, in einem Notbetrieb Parameter der Membranfiltrationsanlage und/oder die Wasserausbeute anzuwenden.

Die Parameter sind dabei bevorzugt Regelparameter und/oder Sollwerte und/oder Prozessvariablen und/oder Wasserausbeute. Die Steuer- und Regeleinheit führt bevorzugt mit Hilfe dieser Parameter Fehler-/Alarm-/ Hinweishandling (d. h. Aktionen, welche durch Ereignisse hervorgerufen werden), Betriebseigenschaften (Reihenfolge Pumpenstart/-stopp) und/oder Interaktionen mit angeschlossenen Peripheriegeräten aus.

Bei mehrstufigen Anlagen können unterschiedliche Parameter für jede Stufe angewendet werden. Bei einem Notbetrieb müssen, in Abhängigkeit des Notbetriebes, die Parameter der defekten Stufe teilweise oder vollständig auf die Kompensationsstufe angewendet werden, um den Prozess qualitativ gleichwertig zu halten. Ebenfalls kann es sein, dass die Steuer- und Regeleinheit andere Sensoren und Aktoren ansteuert, da sich der hydraulische Ablauf innerhalb des Notbetriebes geändert hat.

Das Anpassen bzw. Anwenden der Parameter dient dazu, den Überbrückungsmodus prozessicher durchführen zu können. Es müssen beispielsweise Berechnungen (Regler/Stellgradberechnung) angepasst werden, um sicherzustellen, dass die korrekte/angepasste Prozessvariable herangezogen wird und das richtige Systemverhalten erzielt wird und auch dass die neue Anlagenkonfiguration (z.B: Qualität des Prozesseingangswassers) berücksichtigt wird.

In einer bevorzugten Ausführungsform ist wenigstens ein Umgehungsventil als Magnetventil ausgebildet. Dies entspricht einer kostengünstigen Ausführung der Ventile. Ebenfalls bietet dieses Ventil durch die Funktionsweise keinen undefinierten Zustand, wie ein teilweise geöffnetes Ventil, wie dies beispielsweise bei Scheibenventilen oder elektrischen Stellventilen, bei denen eine Zwischenstellung möglich ist, der Fall wäre. Als Alternative können Regelventile oder Motorsteuerventile verwendet werden, welche sich durch Ansteuerung teilweise- oder vollständig öffnen.

Die jeweilige Verbindungsleitung verbindet einen hydraulischen Ausgang einer Stufe mit einem Eingang der hydraulisch nachfolgenden Stufe, wobei vorteilhafterweise in wenigstens einem Ausgang einer Stufe ein Rückschlagventil angeordnet ist, welches den Rückfluss in diese Stufe verhindert. In einer bevorzugten Ausführungsform weist jeder dieser Stufenausgänge ein Rückschlagventil auf.

Die Steuer- und Regeleinheit ist vorzugsweise konfiguriert, vor einem regulären Betrieb der Membranfiltrationsanlage die Umgehungsventile und/oder Sensoren in einem Testmodus auf ihre Funktionsfähigkeit zu überprüfen. Auf diese Weise können mögliche Defekte oder Funktionsstörungen schon vor dem regulären Betrieb der Anlage erkannt und behoben werden. Dadurch kann die Wahrscheinlichkeit von Störungen im laufen Betrieb der Anlage reduziert werden, wodurch bei einer Dialyse die unterbrechungsfreie Versorgung der Patienten gewährleistet werden kann.

Eine bevorzugte Ausführungsform der Membranfiltrationsanlage umfasst genau zwei Stufen.

In Bezug auf das Verfahren wird die oben genannte Aufgabe erfindungsgemäß dadurch gelöst, dass der Betrieb der Membranfiltrationsanlage im Wesentlichen kontinuierlich von einer Steuer- und Regeleinheit überwacht wird, und wobei bei einer festgestellten Fehlfunktion einer Stufe durch die Steuer- und Regeleinheit wenigstens ein in einer Umgehungsleitung geschaltetes Umgehungsventil öffnet, wodurch eine hydraulische Umgehung dieser Stufe bereitgestellt wird
Die Überbrückung erfolgt dabei bevorzugt durch das Öffnen in Umgehungsleitungen, sodass die Flüssigkeit um die jeweilige Stufe mit Fehlfunktion umgeleitet wird.

Das Festellen einer Fehlfunktion erfolgt bevorzugt durch die Überwachung von Sensoren, wie sie oben im Zusammenhang mit der Membranfiltrationsanlage diskutiert wurden. Die Umschaltung in den Notbetrieb erfolgt automatisch über die integrierte Steuerung bzw. Steuer- und Regeleinheit, es ist keine manuelle Nutzerinteraktion erforderlich. Vorteilhafterweise wird eine kontinuierliche und/oder diskontinuirliche Überwachung von Anlagenkomponenten der Membranfiltrationsanlage durchgeführt, diesen Zustand der Fehlfunktion zu detektieren.

Die Bypassleitungen bzw. Umgehungsleitungen dienen ausschließlich
der Überbrückung ausgefallener Komponenten. Ein gleichzeitiges Schalten beider Bypassleitungen wird bevorzugt verhindert, sodass kein Prozesseingangswasser in den Ringleitungsvorlauf kommt, da dies zu Patientenschädigung führen kann.

Die Vorteile der Erfindung liegen insbesondere darin, dass durch eine automatische Umgehung einer als defekt erkannten Stufe schnell auf die neue Situation reagiert werden, sodass eine kontinuierliche Versorgung der Verbraucher erzielt werden kann. Dies ist insbesondere bei der Verwendung der Membranfiltrationsanlage zur Umkehrosmose in der Dialyse relevant, da eine ungenügende Versorgung die Patienten gesundheitlich gefährden kann.

Ein Ausführungsbeispiel der Erfindung wird anhand einer Zeichnung näher erläutert. Darin zeigen in stark schematisierter Darstellung:
- FIG. 1: eine mehrstufige Membranfiltrationsanlage nach dem Stand der Technik;
- FIG. 2: eine zweistufige Membranfiltrationsanlage in einer ersten bevorzugten Ausführungsform mit Magnetventilen;
- FIG. 3: eine zweistufige Membranfiltrationsanlage in einer zweiten bevorzugten Ausführungsform mit einem Mehrwegeventil;
- FIG. 4: Konfigurationen des Mehrwegeventils der Membranfiltrationsanlage gemäß FIG. 3;
- FIG. 5: eine zweistufige Membranfiltrationsanlage mit mehr als zwei Stufen;
- FIG. 6: eine Stufe einer Membranfiltrationsanlage, und
- FIG. 7: eine erste Stufe einer Membranfiltrationsanlage mit Sensoren.

Gleiche Teile sind in allen Figuren mit denselben Bezugszeichen versehen.

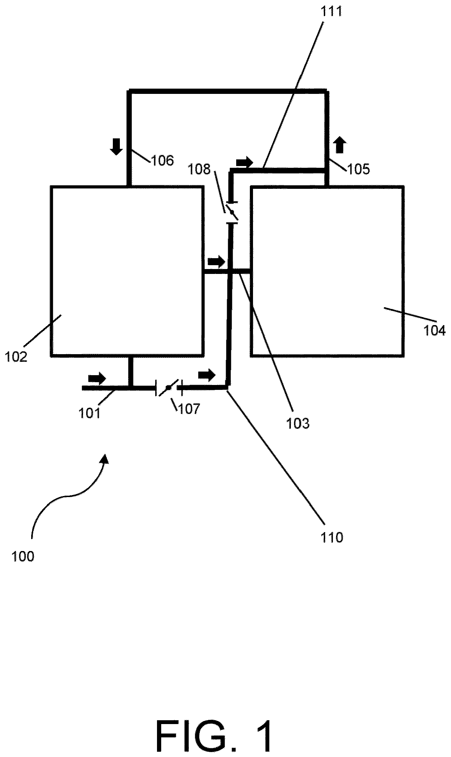

Eine Membranfiltrationsanlage 100 aus dem Stand der Technik ist in FIG. 1 dargestellt. Regular strömt das Prozesseingangswasser 101 in die erste Stufe 102 der Membranfiltrationsanlage 100. Diese Stufe 102 besteht mindestens aus einer Pumpe und einer Filtermembran. Das in dieser Stufe 102 erzeugte Permeat wird über eine Verbindungsstrecke bzw. Verbindungsleitung 103 in die zweite Stufe 104 geleitet. Diese besteht ebenfalls aus mindestens einer Pumpe und einer Filtermembran. Nach der zweiten Filtration wird das Permeat in den Ringleitungsvorlauf 105 geleitet. Wasser, welches nicht aus dem Kreislauf entnommen wird, wird über den Ringleitungsrücklauf 106 zurück in das System geleitet. Bei einer Störung der ersten Stufe 102 kann, sofern die zweite Stufe 104 intakt ist, ein Ventil 107 manuell geöffnet werden, welches das Eingangsprozesswasser 101 durch eine Umgehungsleitung 110 in die zweite Stufe 104 leitet.

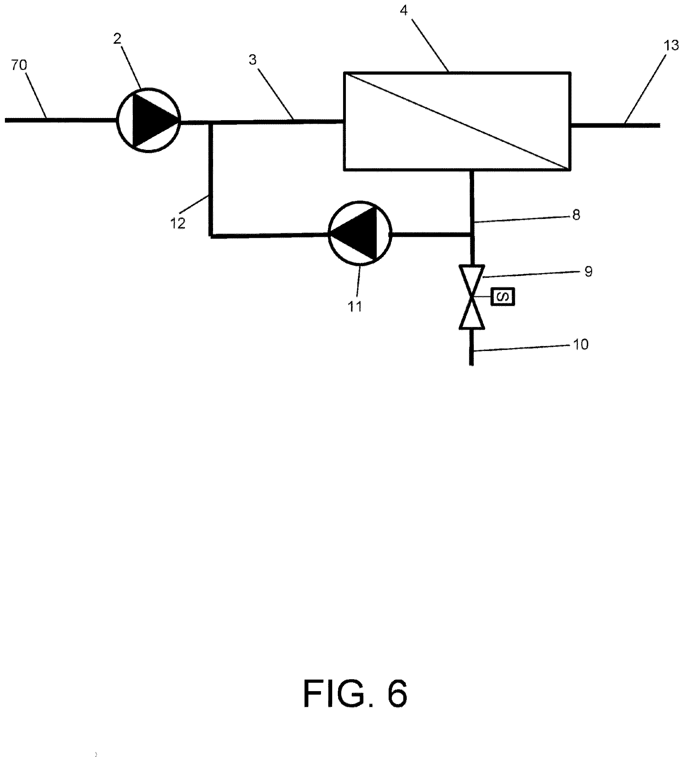

Der bevorzugte hydraulische Aufbau einer Stufe 102, 104 der in FIG. 1 dargestellten Membranfilteranlage 100 ist in FIG. 6 dargestellt. Ein Zulaufstrom 70 wird über eine Druckerhöhungspumpe 2 in ein Membranmodul 4 gepresst. Das Membranmodul 4 kann dabei eine oder mehrere Membranen, die jeweils in Gruppen in Reihe oder parallelgeschaltet sind, enthalten. Des Weiteren können mehrere Membranmodule 4 in Reihe zueinander geschaltet werden.

Durch den Prozess entstehen ausgehend von dem Membranmodul 4 in einer Permeatleitung 13 ein filtrierter Permeatstrom und in einer Konzentratleitung 8 ein Konzentratstrom, welcher die zurückgehaltenen Stoffe abtransportiert. Eine Zirkulationspumpe 11 fördert einen Teil des Konzentrats, d. h. das rezirkulierte Konzentrat 12, im Konzentratstroms zurück zur Druckerhöhungspumpe 2, wo es sich mit dem Zulaufstrom 70 vermischt und als Feedwasser den Prozess wiederholt. Der nicht rezirkulierte Anteil des Konzentrats wird über ein Ventil 9 (z. B. ein Magnetventil, Nadelventil, elektrisches Stellventil, o. Ä.) in die hydraulisch nachfolgende Stufe bzw. in der letzten Stufe als Abwasser 10 aus der Membranfiltrationsanlage 100 geleitet.

Bei einer Störung der zweiten Stufe 104 kann, sofern die erste Stufe 102 intakt ist, ein Ventil 108 manuell geöffnet werden, sodass das Permeat durch eine Umgehungsleitung 111 in den Ringleitungsvorlauf 105 fließen kann . Die Membranfiltrationsanlage 100 produziert während dieser Periode einfach- anstelle von mehrfach gefiltertem Permeat. Das Produkt wird jeweils über den Vorlauf 105 in die Ringleitung eingespeist. Nachteilig bei dieser Lösung ist, dass die Ventile 107, 108 manuell von Hand betätigt werden müssen. Bis zum Umschalten der Ventile 107, 108 kann eine Versorgung der angeschlossenen Verbraucher nicht gewährleistet werden. Auch wird ein gewisses Maß an Anlagenkenntnis vom Bediener vorausgesetzt. Durch falsche Ventilstellungen kann ungefiltertes Wasser in die Ringleitung strömen, welches z. B. bei der Dialysetherapie zu einer Gefährdung der Patienten führen kann.

Bei Weiterentwicklungen der Membranfiltrationsanlage 100 des Stands kann ein Magnetventil verwendet werden, um die zweite Stufe der Anlage zu überbrücken. Die Überbrückung benötigt eine manuelle Interaktion mit dem Nutzer, und es kann nur der Notbetrieb der ersten Stufe 102 über dieses Verfahren realisiert werden.

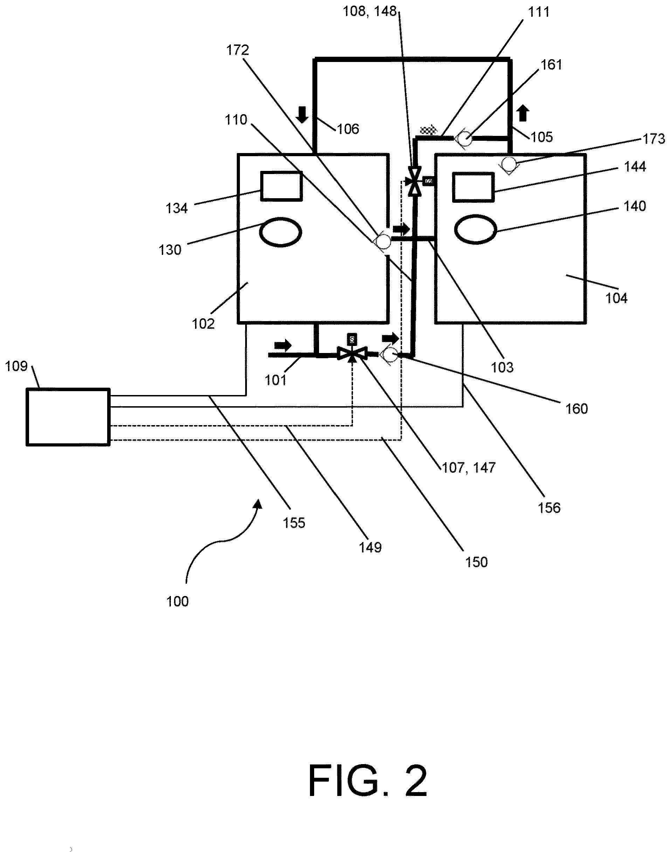

Eine bevorzugte erfindungsgemäße Ausführungsform einer Membranfiltrationsanlage 100 ist in FIG. 2 dargestellt. Auch bei den Ausführungen der Membranfiltrationsanlage 100 gemäß FIG. 2, 3 und 5 ist der bevorzugte hydraulische Aufbau einer Stufe 102, 104 in FIG. 6 dargestellt.

Regular strömt das Prozesseingangswasser 101 in die erste Stufe 102 der Membranfiltrationsanlage 100. Diese Stufe 102 umfasst wenigstens eine Pumpe 130 und wenigstens eine Filtermembran 134. Die Filtermembran 134 kann in einem Membranmodul angeordnet sein. In dem Membranmodul können mehrere Filtermembranen in Reihe und/oder parallel angeordnet sein.

Das in dieser Stufe 102 erzeugte Permeat wird über eine Verbindungsstrecke bzw. Verbindungsleitung 103 in die zweite Stufe 104 geleitet. Diese zweite Stufe 104 umfasst ebenfalls wenigstens eine Pumpe 140 und eine Filtermembran 144. Die Filtermembran 144 kann in einem Membranmodul angeordnet sein. In dem Membranmodul können mehrere Filtermembranen in Reihe und/oder parallel angeordnet sein.

Nach der zweiten Filtration wird das Permeat in den Ringleitungsvorlauf 105 geleitet. Wasser, welches nicht aus dem Kreislauf entnommen wird, gelangt über den Ringleitungsrücklauf 106 zurück in das System. Wie schon im Zusammenhang mit FIG. 1 zum Stand der Technik beschrieben, kann die erste Stufe 102 überbrückt werden, indem das Prozesseingangswasser 101 durch die Umgehungsleitung 110 über ein Ventil 107 zur zweiten Stufe 104 geleitet wird. Die zweite Stufe 104 kann überbrückt werden, indem das Permeat der ersten Stufe 102 durch die Umgehungsleitung 111 über ein Ventil 108 in den Vorlauf 105 geleitet wird. Die beiden Ventile 107, 108 sind vorliegend als Magnetventile 147, 148 ausgeführt.

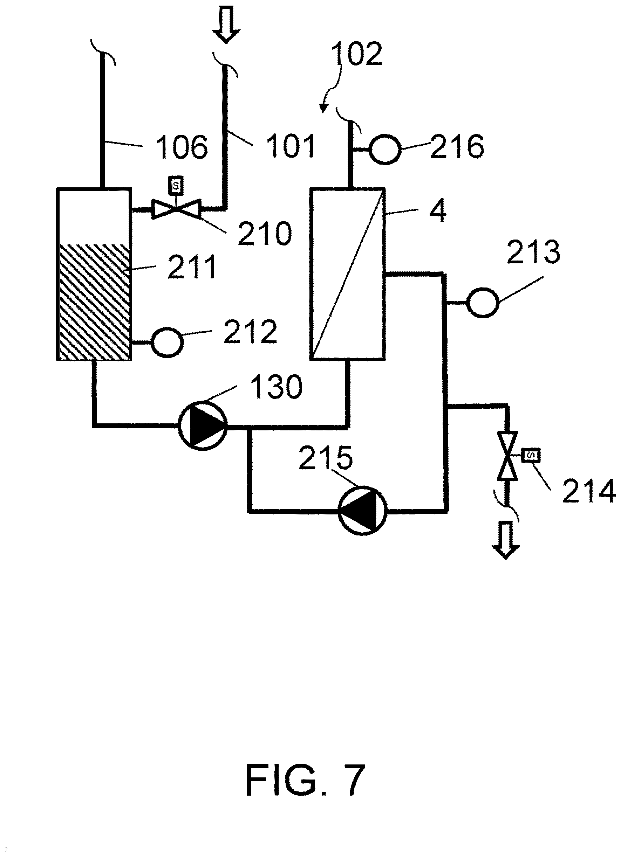

Eine Darstellung der hydraulischen ersten Stufe 102 einer Membranfiltrationsanlage 100 mit Sensoren ist in FIG. 7 dargestellt. Die Stufe 102 weist einen Drucksensor 212 auf, der den Druck im Tank 211 der Membranfiltrationsanlage 100 misst. Ein Volumenstromsensor 213 misst den Konzentratstrom, der aus dem Membranmodul 4 fließt. Ein weiterer Volumenstromsensor 216 misst den aus dem Membranmodul 4 fließenden Permeatstrom.

Das Schalten der Notbetriebsmagnetventile bzw. Magnetventile 147, 148 erfolgt hierbei durch eine Steuer- und Regeleinheit 109. Sie ist dazu mit einer ersten Steuerleitung 149 mit dem Magnetventil 147 und über eine zweite Steuerleitung 150 mit dem zweiten Magnetventil 148 verbunden. Die Steuer- und Regeleinheit 109 ist hardware- und/oder softwaremäßig ausgebildet, insbesondere ist sie eine Kombination aus Software und Hardware und ist eine Komponente der Membranfiltrationsanlage 100.

Die Steuer- und Regeleinheit 109 nutzt zur Beurteilung, ob ein Notbetrieb benötigt wird, Daten aus den jeweiligen Stufen 102, 104. Die Steuer- und Regeleinheit 109 ist dazu signaleingangsseitig über eine erste Signalleitung 155 mit Komponenten der ersten Stufe 102 verbunden und über eine zweite Signalleitung 156 mit Komponenten der zweiten Stufe 104 verbunden. Die beiden Signalleitung 155, 156 sind schematisch eingezeichnet und können mehrere Signalleitungen bzw. einen Bus repräsentieren. Die über die Steuerleitungen 155, 156 an die Steuer- und Regeleinheit 109 übertragenden Daten umfassen Sensorwerte und Prozessdaten. Die Sensorwerte werden von Druck- oder Volumenstromsensoren bzw. Durchflusssensoren (nicht dargestellt) übertragen. Die Prozessdaten werden von Frequenzumrichtern (falls vorhanden) der wenigstens zwei Pumpen 130, 140 und Sicherungsautomaten (nicht dargestellt) übertragen.

Die Steuer- und Regeleinheit 109 vergleicht die übertragenen Sensorwerte bzw. Sensordaten und Prozessdaten mit vorgegebenen Schwellenwerten bzw. Schwellenbereichen. Wenn ein oder mehrere Sensor- oder Prozessdaten außerhalb der Schwellenbereiche liegen bzw. die Schwellenwerte über- bzw. unterschreiten, deaktiviert die Steuer- und Regeleinheit 109 in einem Notbetrieb die als defekt bzw. als nicht funktionsfähig erkannte Stufe 102, 104. Eine Komponente gilt als defekt erkannt, wenn das Über- bzw. Unterschreiten der Schwellenwerte für eine vorgegebene Zeitspanne erfolgt. Eine Stufe 102, 104 gilt als defekt, wenn eine oder mehrere als funktionskritisch deklarierte Komponenten, defekt sind.

Im Folgenden wird beispielhaft die Aktivierung des Notbetriebs durch die Steuer- und Regeleinheit 109 beschrieben. In der Membranfiltrationsanlage 100 können die folgenden Fehler auftreten. Bei einem Sensorfehler ist ein überwachter Sensor defekt und sorgt für die Aktivierung des Notbetriebs. Bei einem Aktorfehler ist ein überwachter Aktor, insbesondere eine Pumpe 130, 140, 215 defekt und aktiviert den Notbetrieb.

Dabei können Verkettungsfehler auftreten. Beispielsweise kann der Notbetrieb durch einen in überwachten Sensor und ein überwachten Aktor durch sie Steuer- und Regeleinheit 109 aktiviert werden. Der Notbetrieb kann auch aktiviert werden durch einen überwachten Sensor, wenn dieser während eines Betriebsmodus (überwacht von der Steuer- und Regeleinheit 109) für einen Zeitintervall unterhalb eines definierten Niveaus liegt.

Ein Sensorfehler liegt beispielhaft vor, wenn das Signal des Tankniveausensors, d.h. das Signal des Drucksensors 212 (siehe FIG. 7) unzuverlässig ist, insbesondere außerhalb eines vorgegebenen Schwellenwertbereiches liegt bzw. der Drucksensor 212 vollständig ausfällt und kein Signal mehr liefert. In diesem Fall ist eine Überwachung des Tankfüllstands des Tanks 211 nicht mehr möglich und die Stufe 102, 104 kann nicht mehr prozesssicher betrieben werden. In diesem Fall schaltet die Steuer- und Regeleinheit 109 das Ventil 107 derart, dass die defekte Stufe 102 überbrückt wird.

Ein Aktorfehler liegt beispielsweise dann vor, wenn die Pumpe 130 defekt ist bzw. die Pumpe 130 oder der optional daran angeschlossene Frequenzumrichter ausgefallen ist. In diesem Fall kann der umkehrosmotische Druck nicht mehr erzeugt werden und die entsprechende Stufe 102 kann kein Permeat erzeugen. Auch in diesem Fall schaltet die Steuer- und Regeleinheit 109 das Ventil 107 derart, dass die defekte Stufe 102 überbrückt wird.

Als Beispiel für einen Verkettungsfehler kann dieser im Zirkulationspfad (Membranmodel 4 über Pumpe 215) der ersten Stufe 102 vorliegen. Die Zirkulationspumpe 215 ist aktiv und der Volumenstromsensor 213 zeigt einen zu geringen Wert (z.B: 0 l/h) an. Die Auswirkung davon ist, dass das entsprechende Membranmodul 4 nicht mehr überströmt wird und es zu einem beschleunigten Verschleiß des Moduls kommt. Ursache dafür können blockierte Leitungsabschnitte oder Probleme mit der Zirkulationspumpe 215 sein.

Bei einem weiteren Beispiel für einen Verkettungsfehler kann die Permeatproduktion der ersten Stufe 102 fehlerhaft sein. Die Membranfiltrationsanlage 100 befindet sich im "Produktions-Modus" (überwacht durch Steuer- und Regeleinheit 109) und der Permeatvolumenstrom der ersten Stufe 102 (erfasst über Volumenstromsensor 216) ist zu gering (z.B: 0 l/h) für einen Zeitraum, der länger ist als ein vorgegebener Zeitraum, beispielsweise 5 Sekunden. Die Auswirkung dieses Fehlers ist, dass die erste Stufe 102 kein Permeat mehr fördert, weil ein Pumpendefekt vorliegt und/oder ein Leitungsdefekt vorliegt und/oder kein Prozesseingangswasser mehr vorhanden ist.

Im beiden Fällen schaltet die Steuer- und Regeleinheit 109 das Ventil 107 derart, dass die defekte Stufe 102 überbrückt wird.

Durch die zentrale Steuer- und Regeleinheit 109 bzw. Steuerungs- und Auswerteeinheit kann somit gewährleistet werden, dass die überbrückte Stufe 102, 104 abgeschaltet wird und die richtigen Ventile 107, 108 geschaltet werden. Ebenfalls kann ein versehentliches Schalten des falschen Ventils 107, 108 bzw. das gleichzeitige Schalten beider Ventile 107, 108 verhindert werden. Die Steuerungs- und Auswerteeinheit 109 ist eine Kombination aus Software und Hardware.

Falls technisch notwendig oder vorteilhaft, werden Rückschlagventile 160, 161 oder funktionell gleichwertige Komponenten Rückschlagklappen, Überströmventile) vor oder hinter die Magnetventile 147, 148 eingebaut, um einen inversen Fluss (aufgrund des Prozessdrucks) zu verhindern. Es ist dann jeweils eine Kombination aus Magnetventil 147, 148 und Rückschlagventil notwendig. Dementsprechend ist ein Rückschlagventil 160 stromaufwärts des Ventils 107 angeordnet, welches den Rückfluss von Flüssigkeit durch das Ventil 107 sperrt. Ein weiteres Rückschlagventil 161 ist stromaufwärts des Ventils 108 angeordnet, welches den Rückfluss von Flüssigkeit durch das Ventil 108 sperrt. Neben dem Auslösen des Notbetriebs innerhalb des laufenden Betriebes kann der Notbetrieb auch schon vor Starten des eigentlichen Betriebes begonnen werden, indem getestet wird ob Sensoren oder Aktoren defekt sind.

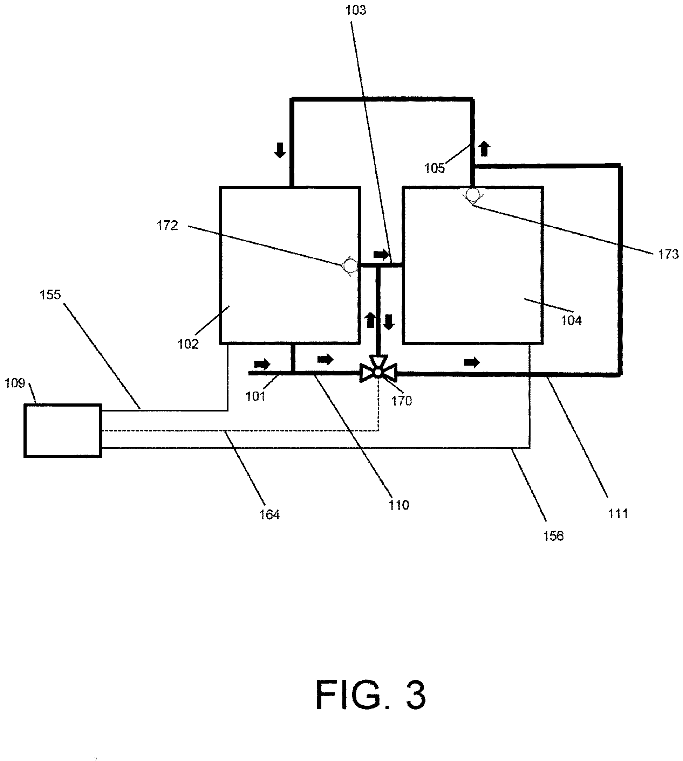

Bei der in FIG. 3 dargestellten Ausführung Variante sind Rückschlagventile vor dem Mehrwegeventil 170 nicht notwendig. In der Regel und bevorzugt besitzen jedoch die einzelnen Stufen 102, 104 jeweils ein Rückschlagventil 172, 173 an ihren Ausgängen, um einen inversen Volumenstrom durch die Stufe zu verhindern.

Eine weitere bevorzugte erfindungsgemäße Ausführungsform einer Membranfiltrationsanlage 100 ist in FIG. 3 dargestellt. Auch bei dieser Ausführungsform umfasst die erste Stufe 102 wenigstens eine Pumpe (nicht dargestellt) und wenigstens eine Filtermembran (nicht dargestellt) und die zweite Stufe 104 umfasst wenigstens eine Pumpe (nicht dargestellt) und eine Filtermembran (nicht dargestellt), wobei jeweils Membranmodule mit in Reihe oder parallelgeschalteten Filtermembranen vorgesehen sein können. Insbesondere sind die beiden Stufen 102, 104 ausgeführt wie im Zusammenhang mit FIG. 6 oben beschrieben, d. h. sie umfassen jeweils eine Druckerhöhungspumpe 2, ein Filtermodul 4 und eine Zirkulationspumpe 11.

Im Unterschied zu der in FIG. 2 dargestellten Ausführungsform sind die Magnetventile 147, 148 durch ein Mehrwegeventil 170 ersetzt, welches von der Steuer- und Regeleinheit 109 über eine Steuerleitung 164 angesteuert wird. Eine unerlaubte Konfiguration dieses Ventils kann zum Beispiel über eine L-Bohrung im Ventil realisiert werden. Das Mehrwegeventil 170 kann die erste Stufe 102 überbrücken, indem das Prozesseingangswasser 101 durch die Umgehungsleitung 110 in das Verbindungsstück 103 bzw. in die zweite Stufe 104 geleitet wird. Die zweite Stufe 104 kann überbrückt werden, indem das Permeat der ersten Stufe am Verbindungsstück 103 über das Mehrwegeventil 170 durch die Umgehungsleitung 111 in den Vorlauf 105 geleitet wird. Der ungültige Pfad, dass das Prozesseingangswasser 101 über das Mehrwegeventil 170 in den Vorlauf 105 geleitet wird, ist durch die Geometrie des Ventils ausgeschlossen.

Auch bei der in FIG. 3 dargestellten Ausführung Variante sind Rückschlagventile vor dem Mehrwegeventil 170 nicht notwendig. In der Regel und bevorzugt besitzen jedoch die einzelnen Stufen 102, 104 jeweils ein Rückschlagventil 172, 173 an ihren Ausgängen, um einen inversen Volumenstrom durch die Stufe zu verhindern.

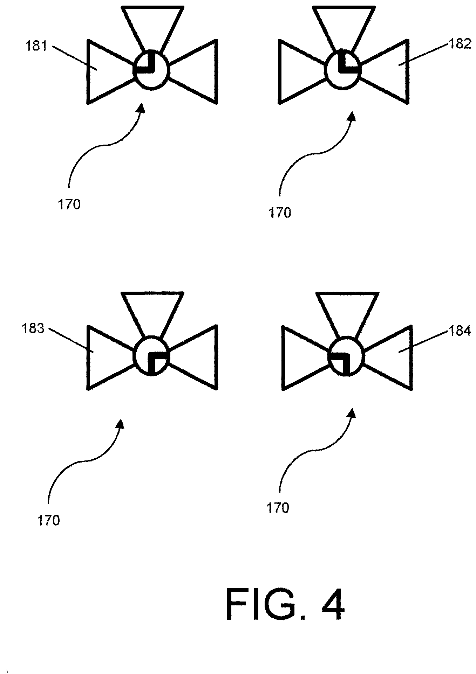

In FIG. 4 sind die exemplarischen Positionen des Mehrwegeventils 170 abgebildet. Hierbei beschreibt die Konfiguration 181 (links oben in FIG. 4) die Überbrückung der ersten Stufe 102, die Konfiguration 182 die Überbrückung der zweiten Stufe 104, und die Konfigurationen 183, 184 bilden jeweils den Normalzustand, also ohne Überbrückung ab.

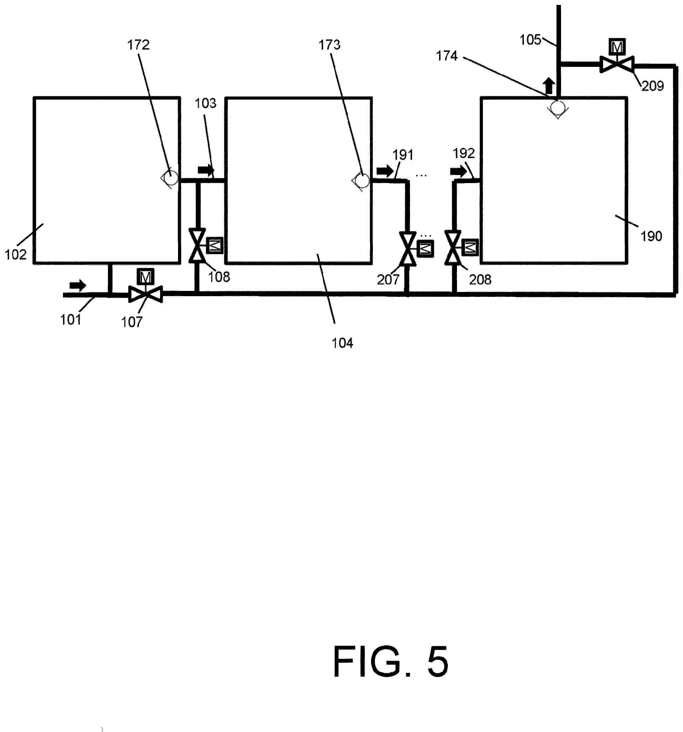

In den FIG. 2 und 3 sind zweistufige Membranfiltrationsanlagen 100 beschrieben. Die erfinderische Idee ist skalierbar auf Membranfiltrationsanlagen 100 mit mehr als zwei Stufen 102, 104. Eine N-stufige (N>2) Anlage ist generisch und schematisch in FIG. 5 dargestellt, wobei die Steuer- und Regeleinheit 109 hier nicht dargestellt ist. Bei dieser N-Stufigen Anlage gelangt im normalen Betrieb das Prozesseingangswasser 101 in die erste Stufe 102. Das Produkt der ersten Stufe 102 wird über eine Verbindungsleitung 103 in die zweite Stufe 104 geleitet. Das generelle Vorgehen wird fortgeführt bis zur N-ten Stufe 190, wonach das Wasser in die Leitung zu dem Vorlauf 105 geleitet wird. Sollte die erste Stufe 102 ausfallen, kann das Prozesseingangswasser 101 über das Ventil 107 zur zweiten Stufe 104 oder alternativ zu einer beliebigen Stufe transportiert werden, indem das korrespondierende Ventil 108, 207 oder 208 geöffnet wird.

Sollte eine Stufe innerhalb des Systems ausfallen, z. B. die zweite Stufe 104, kann diese Stufe 104 überbrückt werden, indem das davor liegende Ventil (hier Ventil 108) und das dahinter liegende Ventil (hier Ventil 207) geöffnet werden. Die defekte Stufe 104 wird somit aus dem System hydraulisch ausgeschlossen. Bei einem Ausfall der letzten Stufe 190 kann über das davorliegende Ventil 208 und das an der Leitung zu den Verbrauchern 220 anliegende Ventil 209 diese Stufe aus dem System hydraulisch ausgeschlossen werden.

Bei der in FIG. 3 dargestellten Ausführung Variante sind Rückschlagventile vor dem Mehrwegeventil 170 nicht notwendig. In der Regel und bevorzugt besitzen jedoch die einzelnen Stufen 102, 104, 190 jeweils ein Rückschlagventil 172, 173 an ihren Ausgängen, um einen inversen Volumenstrom durch die Stufe zu verhindern.

Die Ventile 107, 108, 209, ... können dabei über eine zentrale Steuerungseinheit gesteuert werden. Es können auch anstelle von Motorstellventilen, Magnetventilen o. Ä. auch Mehrwegeventile (siehe FIG. 3) eingesetzt werden.

### Bezugszeichenliste

- 2: Druckerhöhungspumpe
- 4: Membranmodul
- 8: Konzentratleitung
- 9: Ventil
- 11: Zirkulationspumpe
- 12: rezirkuliertes Konzentrat
- 13: Permeatleitung
- 70: Zulaufstrom
- 100: Membranfiltrationsanlage
- 101: Prozesseingangswasser
- 102: erste Stufe
- 103: Verbindungsleitung
- 104: zweite Stufe
- 105: Ringleitungsvorlauf
- 106: Ringleitungsrücklauf
- 107: Ventil
- 108: Ventil
- 110: Umgehungsleitung
- 111: Umgehungsleitung
- 130: Pumpe
- 134: Filtermembran
- 140: Pumpe
- 144: Filtermembran
- 147: Magnetventil
- 148: Magnetventil
- 149: erste Steuerleitung
- 150: zweite Steuerleitung
- 155: erste Signalleitung
- 156: zweite Signalleitung
- 160: Rückschlagventil
- 161: Rückschlagventil
- 164: Steuerleitung
- 170: Mehrwegeventil
- 172: Rückschlagventil
- 173: Rückschlagventil
- 174: Rückschlagventil
- 181: Konfiguration
- 182: Konfiguration
- 183: Konfiguration
- 184: Konfiguration
- 190: N-te Stufe
- 191: Verbindungsleitung
- 192: Verbindungsleitung
- 198: Stufe
- 207: Ventil
- 208: Ventil
- 209: Ventil
- 210: Ventil
- 211: Tank
- 212: Drucksensor
- 213: Volumenstromsensor
- 214: Ventil
- 215: Pumpe
- 216: Volumenstromsensor

## Patentansprüche

1. Membranfiltrationsanlage (100), insbesondere zur Umkehrosmose, umfassend eine Mehrzahl von Stufen (102, 104, 190), wobei jede Stufe (102, 104, 190) wenigstens eine Pumpe und eine Filtermembran umfasst, und wobei die Stufen (102, 104, 190) hydraulisch in Folge durch Verbindungsleitungen (103, 191, 192) hydraulisch miteinander verbunden sind, und wobei für jede Stufe (102, 104, 190) eine Umgehungsleitung (110, 111) zur Umleitung des Flüssigkeitsstroms um diese Stufe (102, 104, 190) vorgesehen ist, und wobei wenigstens ein Umgehungsventil (107, 108, 170, 207, 208, 209) vorgesehen ist, welches im geschlossenen Zustand die jeweilige Umgehungsleitung (110, 111) sperrt,
**dadurch gekennzeichnet, dass**
eine Steuer- und Regeleinheit (109) vorgesehen ist, welche konfiguriert ist, das jeweilige Umgehungsventil (107, 108, 170, 207, 208, 209) anzusteuern.

2. Membranfiltrationsanlage (100) nach Anspruch 1, wobei die Steuer- und Regeleinheit (109) konfiguriert ist, in einem Notbetrieb mit wenigstens einer defekten Stufe (102, 104, 190) das der jeweiligen Stufe (102, 104, 190) zugeordnete Umgehungsventil (107, 108, 170, 207, 208, 209) zu öffnen, sodass die Flüssigkeit um die defekte Stufe (102, 104, 190) herumgeleitet wird.

3. Membranfiltrationsanlage (100) nach Anspruch 1 oder 2, wobei in jeder Umgehungsleitung (110, 111) ein Umgehungsventil (107, 108, 170, 207, 208, 209) angeordnet ist.

4. Membranfiltrationsanlage (100) nach einem der vorherigen Ansprüche, wobei die Steuer- und Regeleinheit (109) konfiguriert ist, signaleingangsseitig Sensordaten und/oder Prozessdaten der Membranfiltrationsanlage (100) zu empfangen.

5. Membranfiltrationsanlage (100) nach Anspruch 4 in Kombination mit Anspruch 2, wobei die Steuer- und Regeleinheit (109) in einem Notbetrieb das der jeweiligen Stufe (102, 104, 190) zugeordnete Umgehungsventil (107, 108, 170, 207, 208, 209) öffnet, sodass die Flüssigkeit um die defekte Stufe (102, 104, 190) herumgeleitet wird, wenn eine die empfangenen Sensordaten und/oder Prozessdaten außerhalb eines vorgegebenen Schwellenbereiches liegen, insbesondere wenn ein Null-Signal eines Sensors vorliegt.

6. Membranfiltrationsanlage (100) nach Anspruch 4 oder 5, wobei die Membranfiltrationsanlage (100) wenigstens einen Drucksensor und/oder wenigstens einen Volumenstromsensor umfasst, wobei der jeweilige Sensor zur Übertragung von Sensordaten signaleingangsseitig mit der Steuer- und Regeleinheit (109) verbunden ist.

7. Membranfiltrationsanlage (100) nach Anspruch 6, wobei der wenigstens eine Drucksensor ein Drucksensor (212) zum Messen des Füllstands eines Tanks (211), insbesondere eines Vorhaltetanks der Membranfiltrationsanlage (100) ist, und/oder wobei der wenigstens eine Volumenstromsensor ein Volumenstromsensor (213) zum Messen des Konzentratstroms ist und/oder wobei der wenigstens eine Volumenstromsensor ein Volumenstromsensor (216) zum Messen des Permeatstroms ist.

8. Membranfiltrationsanlage (100) nach Anspruch 6 oder 7, wobei die Membranfiltrationsanlage (100) wenigstens einen Frequenzumrichter einer Pumpe (130, 140) und/oder wenigstens einen Sicherungsautomat und/oder wenigstens einen Schutzschalter umfasst, welche/r zur Übertragung von Prozessdaten signaleingangsseitig mit der Steuer- und Regeleinheit (109) verbunden ist.

9. Membranfiltrationsanlage (100) nach Anspruch 7 oder 8, wobei die signaleingangsseitige Verbindung analog, digital über elektrische Übertragungsleitungen (155, 156) und/oder über einen Bus erfolgt.

10. Membranfiltrationsanlage (100) nach einem der Ansprüche 6 bis 9, wobei die Steuer- und Regeleinheit (109) bei einem Über- oder Unterschreiten wenigstens eines Schwellenwertes der Sensor- und/oder der Prozessdaten in einem Notbetrieb wenigstens ein Umgehungsventil (107, 108, 170, 207, 208, 209) öffnet.

11. Membranfiltrationsanlage (100) nach Anspruch 10, wobei die Steuer- und Regeleineit (109) im Notbetrieb wenigstens eine Pumpe (2, 130) derart ansteuert, dass weiterhin Prozesseingangswasser durch wenigstens ein intaktes Membranmodul (4) gepresst wird.

12. Membranfiltrationsanlage (100) nach einem der vorherigen Ansprüche, wobei die Steuer- und Regeleinheit (109) konfiguriert ist, die Komponenten der Membranfiltrationsanlage, welche kontinuierliche Signale ausgeben, insbesondere Drucksensoren und/oder Volumenstromsensoren, dauerhaft zu überwachen und die Komponenten der Membranfiltrationsanlage (100), welche keine kontinuierlichen Signale ausgeben, insbesondere Magnetventile (147, 148), in einem Komponententest zu prüfen.

13. Membranfiltrationsanlage (100) nach einem der vorherigen Ansprüche, wobei die Steuer- und Regeleinheit (109) konfiguriert ist, in einem Notbetrieb Parameter der Membranfiltrationsanlage (100) anzuwenden.

14. Membranfiltrationsanlage (100) nach Anspruch 13, wobei die Parameter Regelparameter und/oder Sollwerte und/oder Prozessvariablen und/oder die Wasserausbeute sind.

15. Membranfiltrationsanlage (100) nach einem der vorherigen Ansprüche, wobei wenigstens ein Umgehungsventil (107, 108, 207, 208, 209) als Magnetventil (147, 148) ausgebildet ist.

16. Membranfiltrationsanlage (100) nach einem der vorherigen Ansprüche, wobei die Steuer- und Regeleinheit (109) konfiguriert ist, vor einem regulären Betrieb der Membranfiltrationsanlage (100) die Umgehungsventile (107, 108, 170, 207, 208, 209) und/oder Sensoren in einem Testmodus auf ihre Funktionsfähigkeit zu überprüfen.

17. Membranfiltrationsanlage (100) nach einem der vorherigen Ansprüche, umfassend genau zwei Stufen (102, 104).

18. Verfahren zum Betreiben einer mehrstufigen Membranfiltrationsanlage (100), welche eine Mehrzahl von hydraulisch sich aneinander anschließenden Stufen (102, 104, 190) umfasst, wobei Flüssigkeit nacheinander durch die Stufen (102, 104, 190) geführt wird, in denen sie jeweils gefiltert wird, und wobei das Permeat der jeweils vorherigen Stufe (102, 104) in die hydraulisch jeweils folgende Stufe (104, 190) gefördert wird,
**dadurch gekennzeichnet, dass**
der Betrieb der Membranfiltrationsanlage (100) im Wesentlichen kontinuierlich von einer Steuer- und Regeleinheit (109) überwacht wird, und wobei bei einer festgestellten Fehlfunktion einer Stufe (102, 104, 190) durch die Steuer- und Regeleinheit (109) wenigstens ein in einer Umgehungsleitung (110, 111) geschaltetes Umgehungsventil (107, 108, 170, 207, 208, 209) öffnet, wodurch eine hydraulische Umgehung dieser Stufe (102, 104, 190) bereitgestellt wird.
